# EUROPEAN PATENT APPLICATION

(11) **EP 4 803 006 A1**
(43) Date of publication of application: **09.09.2026**
(21) Application number: 26160431.8
(22) Date of filing: 24.02.2026
(51) Int. Cl.: A61B 6/00

(54) **RADIATION GENERATION APPARATUS, OPERATION METHOD OF RADIATION GENERATION APPARATUS, AND OPERATION PROGRAM OF RADIATION GENERATION APPARATUS**

(30) Priority: 28.02.2025 JP 2025031675
(71) Applicant: FUJIFILM Corporation, Tokyo 106-8620 (JP)
(72) Inventor: HORIUCHI, Hisatsugu, Kanagawa, 258-8538 (JP); WATANABE, Hiroshi, Kanagawa, 258-8538 (JP); KAYASUGA, Atsushi, Kanagawa, 258-8538 (JP); KOBAYASHI, Takeyasu, Kanagawa, 258-8538 (JP); NISHINO, Naoyuki, Kanagawa, 258-8538 (JP)
(74) Representative: Dehns Germany Partnerschaft mbB

(57) **Abstract**

Provided are a radiation generation apparatus, an operation method of a radiation generation apparatus, and an operation program of a radiation generation apparatus that can accurately perform not only driving control to a target position but also alignment control between a radiation source and a radiographic image detection device.

A radiation generation apparatus includes a body part including a radiation source that emits radiation toward a patient, the body part being capable of autonomous driving by a carriage unit having wheels. A driving control unit executes driving control of a carriage unit to a target position based on a surrounding environment and a self-position recognized by a driving control image. An alignment control unit executes alignment control of a radiation source and an electronic cassette based on the electronic cassette recognized by an alignment control image.

## Description

### BACKGROUND OF THE INVENTION

### 1. Field of the Invention

The present disclosed technology relates to a radiation generation apparatus, an operation method of a radiation generation apparatus, and an operation program of a radiation generation apparatus.

### 2. Description of the Related Art

In a medical field, a driving type radiation generation apparatus that can drive by a driving mechanism having wheels, the apparatus including a body part having a radiation source that emits radiation toward a subject, is used. For example, the driving type radiation generation apparatus is used for so-called ward round imaging in which a patient as a subject is imaged while driving around a ward.

WO2017/043040A discloses a manually operated mobile radiation generation apparatus equipped with a camera that images a surrounding environment. In WO2017/043040A, the manual driving to a target position such as a side of a decubitus imaging table (bed) on which an electronic cassette is placed is assisted by notifying an operator such as a radiologic technologist of a path to avoid an obstacle captured by the camera.

### SUMMARY OF THE INVENTION

In a case of using the driving type radiation generation apparatus, first, the driving type radiation generation apparatus is caused to drive to a target position, and then the radiation source and the electronic cassette such as the radiographic image detection device are aligned. For the driving to the target position, for example, autonomous driving using a simultaneous localization and mapping (SLAM) technology is considered. The autonomous driving can reduce a burden on the operator. Even in a case where the autonomous driving is adopted, it is required to accurately align the radiation source and the radiographic image detection device such that the radiation source faces the radiographic image detection device.

One embodiment according to the disclosed technology provides a radiation generation apparatus, an operation method of a radiation generation apparatus, and an operation program of a radiation generation apparatus that can accurately perform not only driving control to a target position but also alignment control between a radiation source and a radiographic image detection device.

A radiation generation apparatus according an aspect of the present invention is a radiation generation apparatus, in which a body part including a radiation source that emits radiation toward a subject is autonomously drivable by a driving mechanism having wheels, the radiation generation apparatus comprising: an environment information sensor; and a processor, in which the processor is configured to execute: driving control of the driving mechanism to a target position based on a surrounding environment and a self-position recognized from output data of the environment information sensor; and alignment control of the radiation source and a radiographic image detection device based on the radiographic image detection device or the subject recognized from the output data.

It is preferable that the processor transitions from the driving control to the alignment control or transitions from the alignment control to the driving control in a case where a set condition is satisfied.

It is preferable that the processor transitions from the driving control to the alignment control in a case where the self-position is the target position.

It is preferable that the processor transitions from the driving control to the alignment control in a case where the radiographic image detection device or the subject is detected from the output data.

It is preferable that the processor transitions from the alignment control to the driving control in a case where a misregistration amount between the radiation source and the radiographic image detection device or the subject is equal to or larger than a set amount.

It is preferable that, in the driving control, map data of the surrounding environment is created while estimating the self-position from the output data.

It is preferable that, in the alignment control, a contour of the radiographic image detection device is extracted from the output data.

It is preferable that, in the alignment control, a trained model trained by output data for training including the radiographic image detection device is used.

It is preferable that, in the alignment control, a joint point of the subject is extracted from the output data.

It is preferable that the body part includes an arm that holds the radiation source and is capable of changing a position and/or a posture of the radiation source, the radiation source is movable between an accommodation position and an imaging preparation position by the arm, and the processor is configured to: set the radiation source to the accommodation position while executing the driving control; and set the radiation source from the accommodation position to the imaging preparation position in a case of transitioning from the driving control to the alignment control.

It is preferable that the alignment control includes control of moving the body part at the target position by the driving mechanism, and a moving speed of the body part in the alignment control is lower than a driving speed of the body part in the driving control.

An operation method of a radiation generation apparatus according to the present disclosed technology is an operation method of a radiation generation apparatus, in which a body part including a radiation source that emits radiation toward a subject is autonomously drivable by a driving mechanism having wheels, the operation method including: executing driving control of the driving mechanism to a target position based on a surrounding environment and a self-position recognized from output data of the environment information sensor; and executing alignment control of the radiation source and a radiographic image detection device based on the radiographic image detection device or the subject recognized from the output data.

An operation program of a radiation generation apparatus according to another aspect of the present invention is an operation program of a radiation generation apparatus, in which a body part including a radiation source that emits radiation toward a subject is autonomously drivable by a driving mechanism having wheels, the operation program causing a computer to execute a process including: executing driving control of the driving mechanism to a target position based on a surrounding environment and a self-position recognized from output data of the environment information sensor; and executing alignment control of the radiation source and a radiographic image detection device based on the radiographic image detection device or the subject recognized from the output data.

According to the disclosed technology, it is possible to provide a radiation generation apparatus, an operation method of a radiation generation apparatus, and an operation program of a radiation generation apparatus that can accurately perform not only driving control to a target position but also alignment control between a radiation source and a radiographic image detection device.

### BRIEF DESCRIPTION OF THE DRAWINGS

FIG. 1 is a diagram showing a state of decubitus imaging using a radiography system.
FIG. 2 is a diagram showing a state of upright imaging using the radiography system.
FIG. 3 is a diagram showing a radiation generation apparatus in which a radiation source is in an accommodation position.
FIG. 4 is a block diagram showing an electric configuration of the radiation generation apparatus.
FIG. 5 is a diagram showing a state in which the radiation generation apparatus drives from a standby position toward a first target position in the decubitus imaging and stops at the first target position.
FIG. 6 is a diagram showing a state in which the radiation generation apparatus drives from a standby position toward a second target position in the upright imaging and stops at the second target position.
FIG. 7 is a diagram showing processing of each processing unit of a processor according to the driving control.
FIG. 8 is a diagram showing processing of each processing unit of the processor according to the driving control.
FIG. 9 is a diagram showing a case where a self-position is the target position, the driving control transitions to the alignment control, and the radiation source is set to the imaging preparation position from the accommodation position.
FIG. 10 is a diagram showing processing of each processing unit of the processor according to the alignment control.
FIG. 11 is a diagram showing processing in a learning phase of a cassette contour extraction model.
FIG. 12 is a diagram showing a state of the alignment control.
FIG. 13 is a flowchart showing an imaging procedure.
FIG. 14 is a diagram showing a case where the electronic cassette is detected from an alignment control image, and the driving control transitions to the alignment control.
FIG. 15 is a diagram showing a case where a patient is detected from the alignment control image, and the driving control transitions to the alignment control.
FIG. 16 is a diagram showing an aspect in which a joint point of the patient is extracted from the aligment control image.
FIG. 17 is a diagram showing a case where a misregistration amount between the radiation source and the electronic cassette or the patient is equal to or larger than a set amount, and the alignment control transitions to the driving control.
FIG. 18 is a diagram showing an aspect in which a moving speed of the body part in the alignment control is lower than a driving speed of the body part in the driving control.

### DESCRIPTION OF THE PREFERRED EMBODIMENTS

### First Embodiment

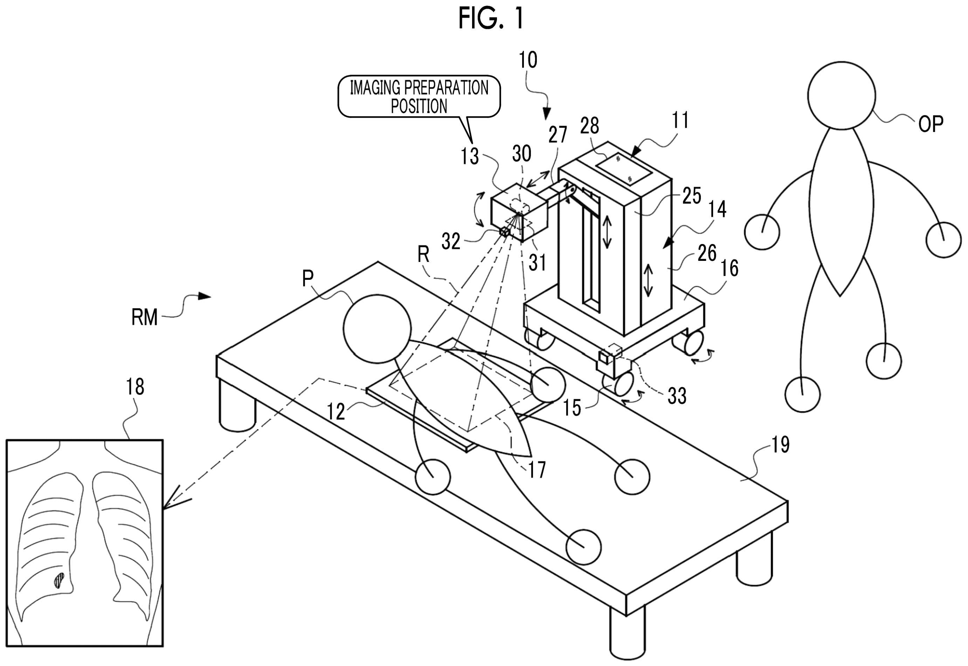

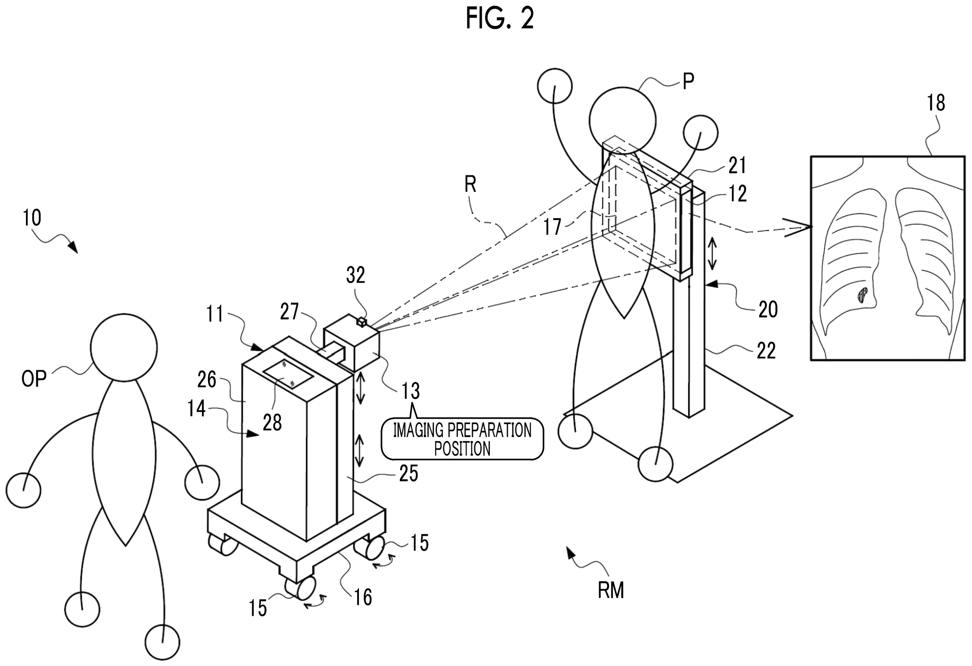

As shown in FIGS. 1 and 2 as an example, a radiography system 10 comprises a radiation generation apparatus 11 and an electronic cassette 12. The radiation generation apparatus 11 has a configuration in which a body part 14 including a radiation source 13 that emits radiation R toward a patient P is mounted on a carriage unit 16 having wheels 15. The radiation R is, for example, an X-ray. A battery is mounted in the radiation generation apparatus 11, and the radiation generation apparatus 11 can be driven by being supplied with power from the battery. In addition, the radiation generation apparatus 11 can also be driven while being supplied with power from a commercial power supply via a power supply cord. Further, the electronic cassette 12 is an example of a "radiographic image detection device" according to the technology of the present disclosure. The carriage unit 16 is an example of a "driving mechanism" according to the disclosed technology. The patient P is an example of a "subject" according to the technology of the present disclosure.

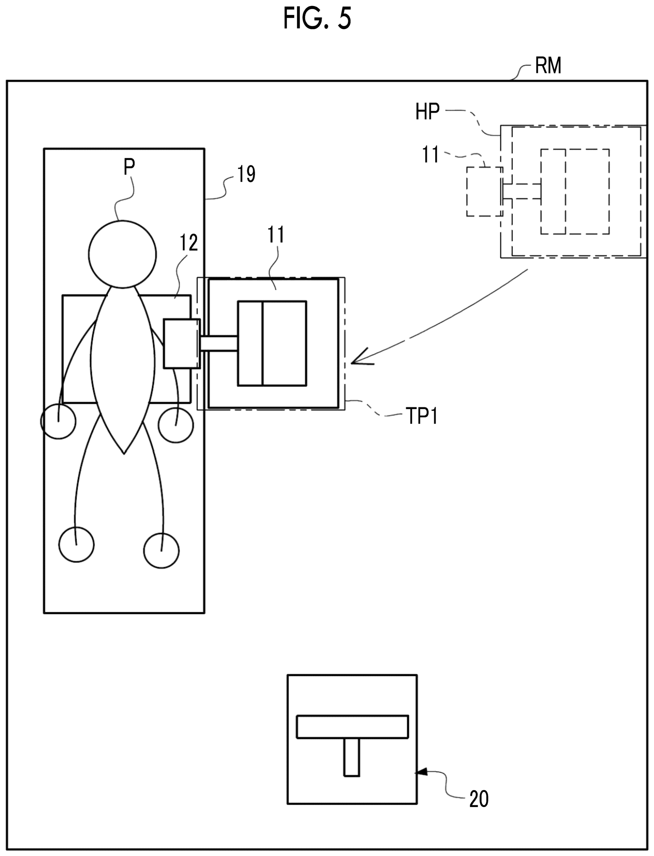

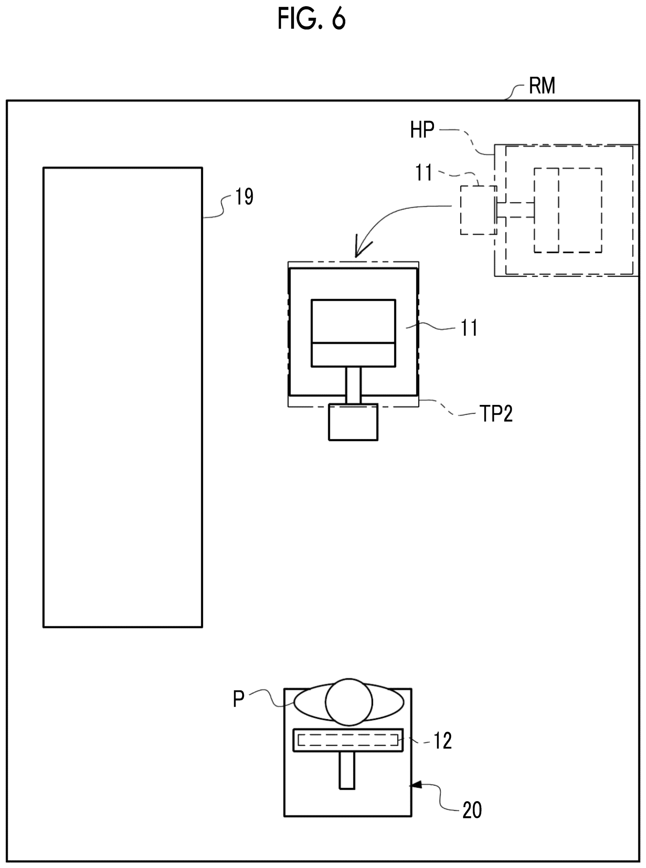

The radiation generation apparatus 11 can move in an imaging room RM (see also FIGS. 5 and 6). In addition, the radiation generation apparatus 11 is used for so-called ward round imaging in which the patient P is imaged while driving around a ward. Therefore, the radiation generation apparatus 11 is also called a ward round cart. Alternatively, the radiation generation apparatus 11 is also used for imaging in an emergency room. In addition, the radiation generation apparatus 11 can be carried into an operating room and used during surgery.

The electronic cassette 12 has a configuration in which a sensor panel as a radiation detector that detects the radiation R is built in a portable housing. The electronic cassette 12 is driven by the battery and performs wireless communication with the radiation generation apparatus 11. The sensor panel has a detection surface 17 in which a plurality of pixels that generate signal charges in response to the radiation R or visible light converted from the radiation R by a scintillator are arranged in a matrix. The electronic cassette 12 detects the radiation R emitted from the radiation source 13 and transmitted through the patient P, and outputs a radiographic image 18 of the patient P.

Since the electronic cassette 12 is portable and wireless, as shown in FIG. 1, the electronic cassette 12 can be used for so-called free imaging in which the electronic cassette 12 is placed on a decubitus imaging table (bed) 19 installed in the imaging room RM (under the patient P) to perform radiography. More specifically, FIG. 1 shows a state in which the electronic cassette 12 is inserted between the decubitus imaging table 19 and the patient P to image the patient P lying on the decubitus imaging table 19 (see also FIG. 5). In addition, as shown in FIG. 2, the electronic cassette 12 can also be used by being accommodated in a holder 21 of an upright imaging table 20 installed in the imaging room RM (see also FIG. 6). The holder 21 can be raised and lowered in the up-down direction with respect to a support column 22.

The body part 14 has a rectangular-parallelepiped shape and is erected at the center of the carriage unit 16. The body part 14 is divided into a front part 25 and a rear part 26. The front part 25 can be raised and lowered in the up-down direction with respect to the rear part 26. The rear part 26 is fixed to the carriage unit 16.

A base end of an arm 27 is attached to the front part 25. More specifically, the arm 27 is divided into a first portion in which the base end is attached to the front part 25 and a second portion in which the base end is attached to the first portion. The radiation source 13 is attached to a distal end of the second portion, which is a free end opposite to the base end.

The first portion can be raised and lowered in the up-down direction with respect to the front part 25 and can be bent in the up-down direction with respect to the front part 25. The second portion can be bent in the up-down direction with respect to the first portion. Further, the second portion is extensible and contractible. The radiation source 13 can rotate with respect to the second portion, that is, can swing. By the displacement of the arm 27 and the displacement of the radiation source 13 with respect to the arm 27, a height position, a horizontal position, and a posture (orientation) of the radiation source 13 can be adjusted.

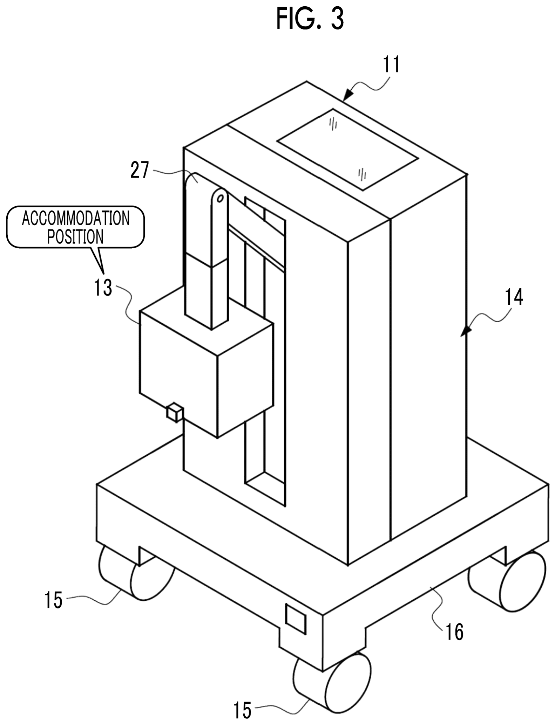

Since the second portion is bendable and extensible and contractible, the radiation source 13 can be moved between an imaging preparation position protruding toward the patient P (electronic cassette 12) as shown in FIGS. 1 and 2 and an accommodation position pulled toward the body part 14 as shown in FIG. 3 as an example. The imaging preparation position is a position in a case where the second portion is extended to a predetermined length, for example, a length of half of the longest length, and the second portion is bent with respect to the first portion to be parallel to the horizontal direction. The accommodation position is a position in a case where the second portion is contracted to the shortest length and the second portion is folded as much as possible with respect to the first portion.

In FIGS. 1 and 2, an operation panel 28 is provided on an upper surface of the rear part 26. The operation panel 28 is configured by, for example, a touch panel display and has a function of displaying information in addition to an operation function. The operation panel 28 is operated by an operator OP such as a radiologic technologist. The operator OP sets an irradiation condition of the radiation R through the operation panel 28. In addition, the operator OP checks the radiographic image 18 through the operation panel 28.

In addition, an irradiation switch (not shown) is provided in the rear part 26. The irradiation switch is a switch that is provided to allow the operator OP to give an instruction to start irradiation of radiation. An extension cable is connected to the irradiation switch, and can be detached from the rear part 26 for use. The irradiation switch can be operated only after driving control and alignment control, which will be described below, are completed.

The radiation source 13 includes a radiation tube 30 and an irradiation field limiter 31. The radiation tube 30 generates the radiation R. The radiation tube 30 is provided with a filament, a target, a grid electrode, and the like (all are not illustrated). A tube voltage is applied between the filament that is a cathode and the target that is an anode from a voltage generator (not shown) built in the rear part 26. The filament releases thermal electrons according to the applied tube voltage toward the target. The target radiates the radiation R in response to collision of the thermoelectrons released from the filament. The grid electrode is disposed between the filament and the target, and changes a flow rate of the thermoelectrons from the filament toward the target in response to the voltage applied from the voltage generator. The flow rate of the thermal electrons from the filament toward the target is referred to as a tube current. The tube voltage and the tube current are set to the radiation source 13 as the irradiation condition over the irradiation time.

The irradiation field limiter 31 is also called a collimeter and limits an irradiation field of the radiation R generated from the radiation tube 30. For example, the irradiation field limiter 31 has a configuration in which four shield plates formed of lead or the like shielding radiation R are disposed on respective sides of a quadrangle, and an emission opening of the quadrangle transmitting radiation is formed in a center portion. The irradiation field limiter 31 changes a size of the emission opening by changing a position of each shielding plate, thereby changing the irradiation field of the radiation R.

A camera 32 is attached to the radiation source 13. The camera 32 is used to support the alignment of the radiation source 13 and the electronic cassette 12. The camera 32 is provided with an imaging element that is sensitive to visible light, for example, a complementary metal oxide semiconductor (CMOS) image sensor or a charge-coupled device (CCD) image sensor. The camera 32 captures a video image at a predetermined frame rate. The camera 32 is an example of an "environment information sensor" according to the disclosed technology.

The wheels 15 are provided in four positions in front, behind, left, and right of the carriage unit 16. That is, the carriage unit 16 is a four-wheel type. Each wheel 15 is, for example, a revolution type that revolves around a revolution axis extending in a height direction (also referred to as a vertical direction) orthogonal to a rotation axis in a case of driving and rotating. The body part 14 autonomously drives by the carriage unit 16.

Here, the autonomous driving refers to autonomously driving toward a set target position while recognizing the surrounding environment using the environment information sensor and estimating the self-position. For example, a position on a side of the decubitus imaging table 19 shown in FIG. 1 and a position facing the upright imaging table 20 shown in FIG. 2 are set in advance as the target position. In the radiation generation apparatus 11, the SLAM technology is used to realize the autonomous driving.

A camera 33 is provided in the carriage unit 16. The camera 33 is used to support the autonomous driving of the body part 14. The camera 33 images a front side of the carriage unit 16. The camera 33 is provided with an imaging element that is sensitive to visible light and captures a video image at a predetermined frame rate, similarly to the camera 32. The camera 33 is also an example of an "environment information sensor" according to the disclosed technology, similarly to the camera 32. It should be noted that the body part 14 and the radiation generation apparatus 11 can also be manually drived by the operator OP, in addition to the autonomous driving.

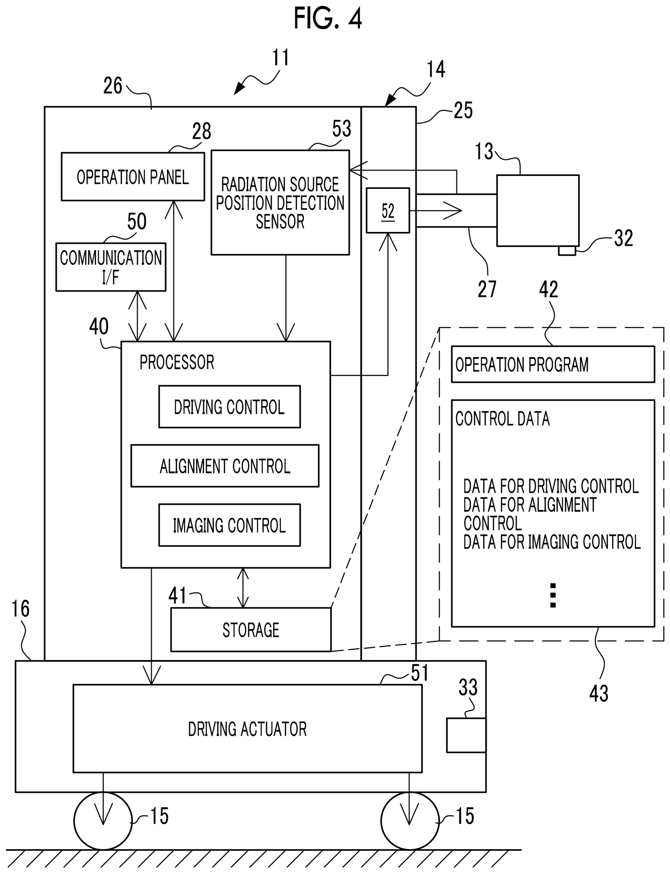

FIG. 4 is a block diagram showing an example of an electric configuration of the radiation generation apparatus 11. A processor 40 integrally controls the entire radiation generation apparatus 11. The processor 40 is configured by, for example, a central processing unit (CPU) and a memory such as a random access memory (RAM), and functions as various processing units by loading and executing various programs in the memory. Specifically, the processor 40 executes driving control, alignment control, and imaging control. The driving control is control related to the autonomous driving of the body part 14. The alignment control is control related to the alignment of the radiation source 13 and the electronic cassette 12. The imaging control includes irradiation control of the radiation R by the radiation source 13, output control of the radiographic image 18 by the electronic cassette 12, and the like.

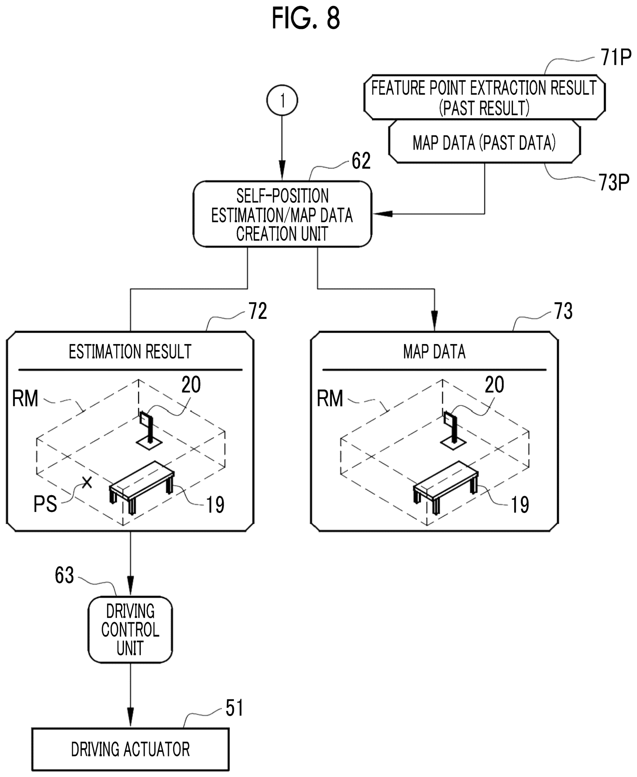

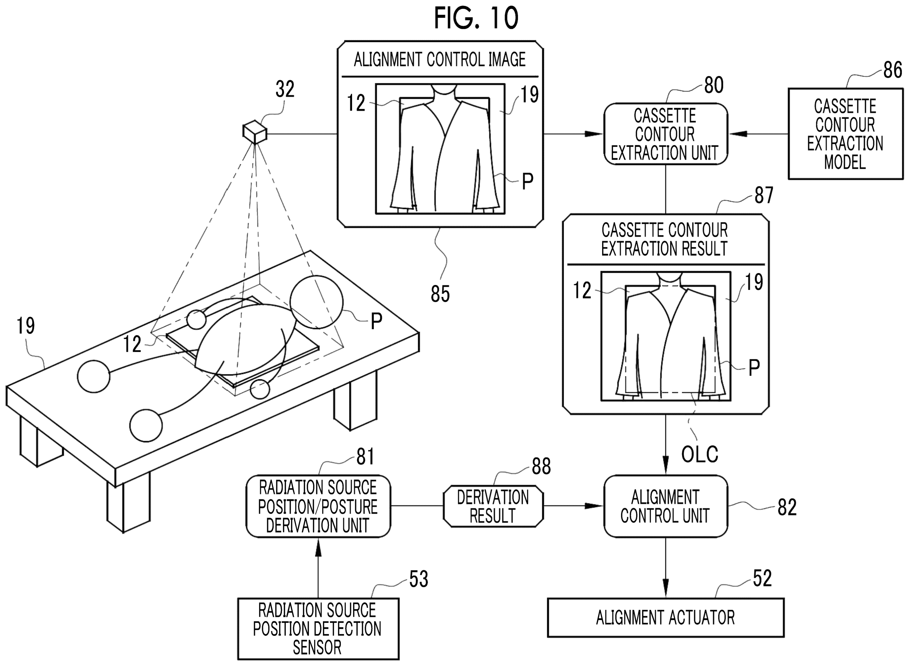

A storage 41 is configured by a non-volatile memory such as a hard disk drive or a solid state drive. The storage 41 stores an operation program 42 and control data 43. The operation program 42 is an example of an "operation program of a radiation generation apparatus" according to the disclosed technology. The control data 43 includes data for driving control, data for alignment control, and data for imaging control. Examples of the data for driving control include map data 73 (see FIG. 8) created by the SLAM technology. Examples of the data for alignment control include a cassette contour extraction model 86 (see FIG. 10) for extracting a contour OLC (see FIG. 10) of the electronic cassette 12. Examples of the data for imaging control include an irradiation condition table in which an irradiation condition for each imaging part is registered.

The operation panel 28, a communication interface (I/F) 50, a driving actuator 51, an alignment actuator 52, and a radiation source position detection sensor 53 are connected to the processor 40. The processor 40 performs display control of various screens on the operation panel 28. In addition, the processor 40 receives various operation instructions of the operator OP through the operation panel 28 and executes various controls in response to the various operation instructions. The communication I/F 50 is, for example, a wireless communication I/F and performs wireless communication with the electronic cassette 12.

The driving actuator 51 includes a motor for driving rotation of the wheels 15 and a motor for revolution under the control of the processor 40. The alignment actuator 52 includes a motor for raising and lowering the front part 25, a motor for raising and lowering the arm 27, a motor for bending the second portion of the arm 27, a motor for extending and contracting the second portion, and a motor for rotating the radiation source 13 with respect to the second portion, under the control of the processor 40.

The radiation source position detection sensor 53 measures an elevation direction and an elevation amount of the arm 27 with respect to the front part 25, a bending direction and a bending amount of the second portion of the arm 27 with respect to the first portion, an extension and contraction direction and an extension and contraction amount of the second portion, and a rotation direction and a rotation amount of the radiation source 13 with respect to the second portion. The radiation source position detection sensor 53 is, for example, a rotary encoder, a potentiometer, a gyro sensor, or a combination of a plurality of types of these sensors. The radiation source position detection sensor 53 outputs the measurement value to the processor 40. The processor 40 derives the position and the posture of the radiation source 13 based on the measurement value of the radiation source position detection sensor 53.

As shown in FIGS. 5 and 6 as an example, a standby position HP of the radiation generation apparatus 11 is prepared in a corner of the imaging room RM. In the standby position HP, the radiation source 13 is in the accommodation position. In the standby position HP, charging of the battery, reception of an imaging order from a radiology information system (RIS), setting of an irradiation condition, and the like are performed. The standby position HP occupies a region equal to, or one size larger than, that of the radiation generation apparatus 11.

In the imaging room RM, a first target position TP1 (see FIG. 5) of the radiation generation apparatus 11 in the decubitus imaging and a second target position TP2 (see FIG. 6) of the radiation generation apparatus 11 in the upright imaging are set. The first target position TP1 is a position on a side of the decubitus imaging table 19, and more specifically, is a position facing one long side of the decubitus imaging table 19. The second target position TP2 is a position facing the upright imaging table 20 and spaced from the upright imaging table 20 by a distance of an SID required for the upright imaging. The first target position TP1 and the second target position TP2 occupy a region equal to, or one size larger than, that of the radiation generation apparatus 11 by one turn, like the standby position HP. It should be noted that, hereinafter, the first target position TP1 and the second target position TP2 may be collectively referred to as a target position TP.

In a case of the decubitus imaging, the radiation generation apparatus 11 drives from the standby position HP toward the first target position TP1 and stops in a case where the self-position PS (see FIG. 8) reaches the first target position TP1 (see FIG. 5). In addition, in a case of the upright imaging, the radiation generation apparatus 11 drives from the standby position HP toward the second target position TP2 and stops in a case where the self-position PS reaches the second target position TP2 (see FIG. 6). Although not shown, the radiation generation apparatus 11 may drive from the first target position TP1 toward the second target position TP2 in order to perform the upright imaging after the decubitus imaging. In addition, the radiation generation apparatus 11 may drive from the second target position TP2 toward the first target position TP1 in order to perform the decubitus imaging after the upright imaging. In a case of the autonomous driving to the target position TP, the radiation source 13 is still in the accommodation position. It should be noted that, in a case where the self-position PS matches the center of the target position TP, it may be determined that the self-position PS has reached the target position TP, or in a case where the self-position PS is within a range including the center of the target position TP with a certain margin, it may be determined that the self-position PS has reached the target position TP.

The autonomous driving to the target position TP is started, for example, by an instruction of the operator OP through the operation panel 28. It should be noted that, instead of or in addition to the operation panel 28, a configuration may be adopted in which an instruction to start the autonomous driving can be issued by a remote controller.

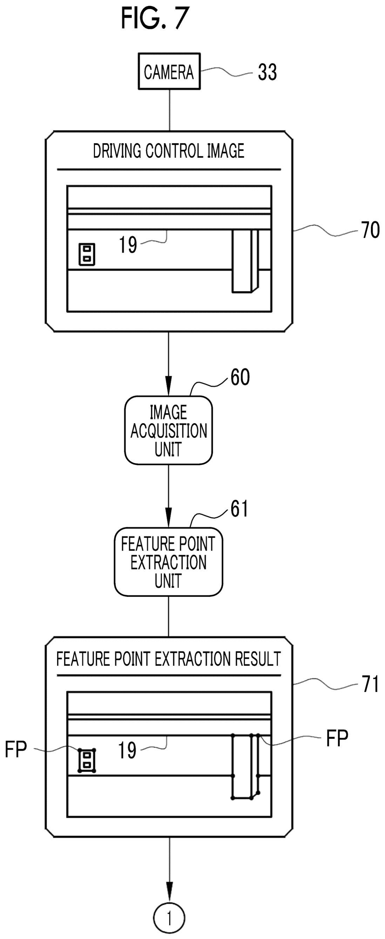

The processor 40 executes the driving control using the SLAM technology. Specifically, as shown in FIGS. 7 and 8, the processor 40 functions as an image acquisition unit 60, a feature point extraction unit 61, a self-position estimation/map data creation unit 62, and a driving control unit 63.

The image acquisition unit 60 sequentially acquires an image 70 (hereinafter, referred to as a driving control image) of the surrounding environment of the radiation generation apparatus 11 captured by the camera 33 at a predetermined frame rate. The image acquisition unit 60 performs preprocessing such as noise removal and distortion correction on the driving control image 70, and then outputs the driving control image 70 to the feature point extraction unit 61. The driving control image 70 is an example of "output data" according to the disclosed technology.

The feature point extraction unit 61 extracts a corner of a structure present in the surrounding environment shown in the driving control image 70 as a feature point FP by using an algorithm such as oriented features from accelerated segment test and rotated binary robust independent elementary features (ORB) or speeded-up robust features (SURF). The feature point extraction unit 61 outputs a feature point extraction result 71 to the self-position estimation/map data creation unit 62. In addition, although not shown, the feature point extraction unit 61 stores the feature point extraction result 71 in the storage 41. The feature point extraction result 71 is a set of coordinates of each feature point FP and a feature amount vector.

The self-position estimation/map data creation unit 62 estimates the self-position PS of the radiation generation apparatus 11 and creates the map data 73 of the surrounding environment. The feature point extraction result 71 is input to the self-position estimation/map data creation unit 62 from the feature point extraction unit 61. In addition, a feature point extraction result (hereinafter, referred to as a feature point extraction result (past result)) 71P for a plurality of past frames and map data (hereinafter, referred to as map data (past data)) 73P for a plurality of past frames are input to the self-position estimation/map data creation unit 62.

The feature point extraction result (past result) 71P and the map data (past data) 73P are stored in the storage 41 as the data for driving control of the control data 43. The feature point extraction result 71 and the map data 73 constituting the feature point extraction result (past result) 71P and the map data (past data) 73P are, for example, for several tens to several hundreds of frames. The feature point extraction result (past result) 71P and the map data (past data) 73P include the feature point extraction result 71 and the map data 73 that are considered to play an important role in the estimation of the self-position PS and the creation of the map data 73. The feature point extraction result 71 and the map data 73 are, for example, the feature point extraction result 71 and the map data 73 obtained for each movement of a certain distance. In addition, for example, the feature point extraction result 71 and the map data 73 obtained in a case where a large viewpoint change has occurred from the previous frame. Further, for example, the feature point extraction result 71 and the map data 73 obtained in a case where a set amount or more of new feature points FP are extracted.

The self-position estimation/map data creation unit 62 matches the feature point FP of the feature point extraction result 71 from the feature point extraction unit 61 with the feature point FP of the feature point extraction result (past result) 71P. In this case, the self-position estimation/map data creation unit 62 refers to the feature amount vector of each feature point FP. More specifically, the feature point extraction unit 61 recognizes the feature points FP having a distance of the feature amount vector (Euclidean distance or the like) less than a threshold value between the feature point FP of the feature point extraction result 71 from the feature point extraction unit 61 and the feature point FP of the feature point extraction result (past result) 71P as the same feature point FP. The self-position estimation/map data creation unit 62 estimates the self-position PS based on the matching result of the feature point FP and the map data (past data) 73P. In addition, the self-position estimation/map data creation unit 62 creates (updates the map data 73) the new map data 73 based on the feature point extraction result 71 from the feature point extraction unit 61, the feature point extraction result (past result) 71P, the map data (past data) 73P, and an estimation result 72. As described above, the self-position estimation/map data creation unit 62 estimates the self-position PS and creates the map data 73 in parallel in a process in which the radiation generation apparatus 11 autonomously drives. It should be noted that the self-position estimation/map data creation unit 62 does not estimate the self-position PS until a sufficient amount of the feature point extraction result (past result) 71P is accumulated, and only creates (updates) the map data 73.

The self-position estimation/map data creation unit 62 outputs the estimation result 72 of the self-position PS to the driving control unit 63. The driving control unit 63 controls the driving of the driving actuator 51 such that the self-position PS is the target position TP. In addition, although not shown, the self-position estimation/map data creation unit 62 stores the map data 73 in the storage 41. Specifically, the map data 73 is three-dimensional data of the imaging room RM including a structure such as the decubitus imaging table 19 and the upright imaging table 20. In addition, the standby position HP and the target position TP are registered in the map data 73.

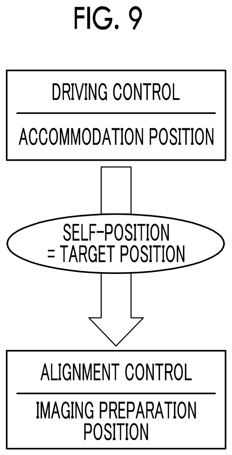

In a case where the driving actuator 51 is driven under the control of the driving control unit 63 and the self-position PS of the radiation generation apparatus 11 is the target position TP, as shown in FIG. 9 as an example, the processor 40 transitions from the driving control to the alignment control. In a case where the self-position PS of the radiation generation apparatus 11 is the target position TP, it is an example of a "case where a set condition is satisfied" according to the disclosed technology. In addition, in a case where the self-position PS of the radiation generation apparatus 11 is the target position TP, the processor 40 sets the radiation source 13 to the imaging preparation position from the accommodation position.

As shown in FIG. 10 as an example, the processor 40 functions as a cassette contour extraction unit 80, a radiation source position/posture derivation unit 81, and an alignment control unit 82.

An image 85 (hereinafter, referred to as an alignment control image) including the patient P and the electronic cassette 12 captured by the camera 32 at a predetermined frame rate is sequentially input to the cassette contour extraction unit 80. In a case where the self-position PS of the radiation generation apparatus 11 is the target position TP and the radiation source 13 is the imaging preparation position, the patient P and the electronic cassette 12 are shown in the alignment control image 85. In FIG. 10, since the decubitus imaging is shown as an example, the decubitus imaging table 19 is also shown in the alignment control image 85. The alignment control image 85 is an example of "output data" according to the disclosed technology. It should be noted that the alignment of the patient P and the electronic cassette 12 is performed by the operator OP before the driving control and the alignment control.

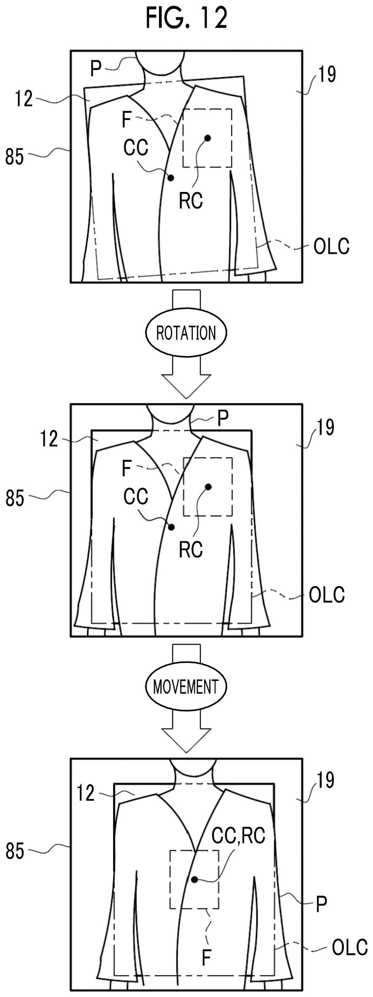

The cassette contour extraction unit 80 extracts the contour OLC of the electronic cassette 12 from the alignment control image 85 by using a cassette contour extraction model 86. According to the contour OLC, the center CC (see FIG. 12) of the detection surface 17 of the electronic cassette 12 and the posture of the electronic cassette 12 with respect to the radiation source 13 are known. The cassette contour extraction unit 80 outputs a cassette contour extraction result 87 to the alignment control unit 82. It should be noted that, here, the contour following all sides of the electronic cassette 12 is shown as the contour OLC, but the present disclosure is not limited to this. Four corners of the electronic cassette 12 may be extracted as the contour.

The cassette contour extraction model 86 is a trained model that is configured by, for example, a convolutional neural network and the like, and that is trained to output the cassette contour extraction result 87 in a case where the alignment control image 85 is input. The cassette contour extraction model 86 is stored in the storage 41 as the data for alignment control of the control data 43.

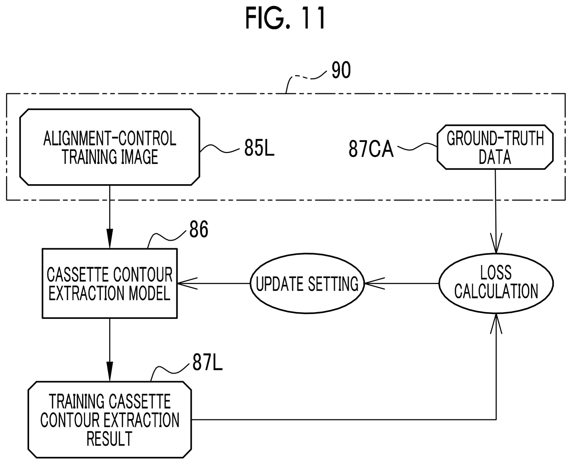

As shown in FIG. 11 as an example, in the learning phase, learning data 90 is given to the cassette contour extraction model 86. The learning data 90 is composed of a set of an alignment-control training image 85L and ground-truth data 87CA. The electronic cassette 12 is shown in the alignment-control training image 85L. The ground-truth data 87CA is data in which the contour OLC of the electronic cassette 12 shown in the alignment-control training image 85L is annotated, and is data for checking the answer. The alignment-control training image 85L is an example of "output data for training" according to the disclosed technology.

The alignment-control training image 85L is input to the cassette contour extraction model 86. The cassette contour extraction model 86 outputs a training cassette contour extraction result 87L in response to the input of the alignment-control training image 85L. The loss calculation of the cassette contour extraction model 86 using the loss function is performed based on the training cassette contour extraction result 87L and the ground-truth data 87CA. Then, the update setting of various coefficients (coefficients of a filter of a convolutional layer and the like) of the cassette contour extraction model 86 is performed according to the result of the loss calculation, and the cassette contour extraction model 86 is updated according to the update setting.

In the learning phase of the cassette contour extraction model 86, the series of processing of the input of the alignment-control training image 85L to the cassette contour extraction model 86, the output of the training cassette contour extraction result 87L from the cassette contour extraction model 86, the loss calculation, the update setting, and the update of the cassette contour extraction model 86 is repeatedly performed while the learning data 90 is replaced. The repetition of the series of processing is ended in a case where the extraction accuracy of the training cassette contour extraction result 87L reaches a predetermined set level. The cassette contour extraction model 86 in which the extraction accuracy reaches the set level is stored in the storage 41 and is used by the cassette contour extraction unit 80. It should be noted that the learning may be ended in a case where the series of processing is repeated a set number of times regardless of the extraction accuracy of the training cassette contour extraction result 87L.

The radiation source position/posture derivation unit 81 derives the position and the posture of the radiation source 13 based on the measurement value of the radiation source position detection sensor 53. The radiation source position/posture derivation unit 81 outputs a derivation result 88 to the alignment control unit 82. The derivation result 88 is coordinates of an irradiation center RC (see FIG. 12) of the radiation R in the alignment control image 85 and coordinates of a rectangular frame F (see FIG. 12) indicating the posture of the radiation source 13 in the alignment control image 85. Since the positional relationship between the radiation source 13 and the camera 32 is known, the coordinates of the irradiation center RC and the frame F in the alignment control image 85 can be easily calculated from the measurement value of the radiation source position detection sensor 53.

The alignment control unit 82 controls the driving of the alignment actuator 52 such that the radiation source 13 and the electronic cassette 12 face each other. More specifically, as shown in FIG. 12 as an example, in a case where the posture of the radiation source 13 is inclined with respect to the electronic cassette 12, the alignment control unit 82 rotates the radiation source 13 to eliminate the inclination. In addition, in a case where the irradiation center RC of the radiation R and the center CC of the detection surface 17 of the electronic cassette 12 are shifted, the alignment control unit 82 moves the radiation source 13 to eliminate the shift. It should be noted that the inclination may be eliminated after the shift between the irradiation center RC of the radiation R and the center CC of the detection surface 17 of the electronic cassette 12 is eliminated. In addition, in FIG. 12, a case where the posture of the radiation source 13 is inclined around the normal line of the detection surface 17 is shown as an example, but the present disclosure is not limited to this. Even in a case where the posture of the radiation source 13 is inclined around an axis along the long side or around an axis along the short side of the detection surface 17, the alignment control unit 82 rotates the radiation source 13 to eliminate the inclination.

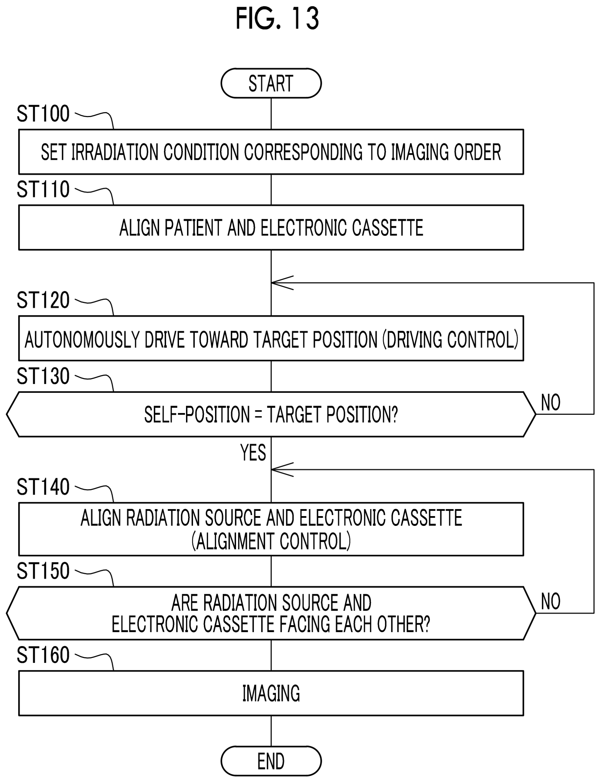

Next, an action with the configuration described above will be described with reference to the flowchart shown in FIG. 13 as an example. Before the imaging, the radiation generation apparatus 11 is on standby at the standby position HP in the imaging room RM. In this case, the radiation source 13 is in the accommodation position shown in FIG. 3.

An imaging order is transmitted from the radiology information system to the radiation generation apparatus 11. The operator OP operates the operation panel 28 to set the irradiation condition corresponding to the imaging order (step ST100).

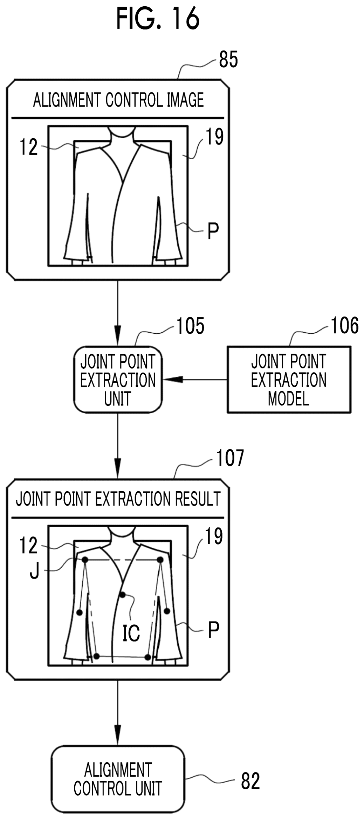

The operator OP places the electronic cassette 12 on the decubitus imaging table 19 in a case of the decubitus imaging, and accommodates the electronic cassette 12 in the holder 21 of the upright imaging table 20 in a case of the upright imaging. Then, the patient P and the electronic cassette 12 are aligned (step ST110). Specifically, the center IC (see FIG. 16) of the imaging part of the patient P and the center CC of the detection surface 17 of the electronic cassette 12 are aligned. In addition, the body axis (head-tail axis) of the patient P and the long side of the detection surface 17 of the electronic cassette 12 are made parallel to each other.

The autonomous driving of the radiation generation apparatus 11 from the standby position HP toward the target position TP is started in response to the instruction of the operator OP (step ST120). In the autonomous driving, the processor 40 executes the driving control using the SLAM technology, which is shown in FIGS. 7 and 8 as an example.

Specifically, first, the driving control image 70 captured by the camera 33 is acquired by the image acquisition unit 60. The driving control image 70 is output from the image acquisition unit 60 to the feature point extraction unit 61. Then, in the feature point extraction unit 61, the feature point FP of the structure present in the surrounding environment shown in the driving control image 70 is extracted. The feature point extraction result 71 is output from the feature point extraction unit 61 to the self-position estimation/map data creation unit 62. In addition, the feature point extraction result 71 is stored in the storage 41.

In the self-position estimation/map data creation unit 62, the self-position PS of the radiation generation apparatus 11 is estimated based on the feature point extraction result 71, the feature point extraction result (past result) 71P, and the map data (past data) 73P, and the estimation result 72 is output. In addition, in the self-position estimation/map data creation unit 62, the map data 73 is created based on the feature point extraction result 71, the feature point extraction result (past result) 71P, the map data (past data) 73P, and the estimation result 72. The estimation result 72 of the self-position PS is output from the self-position estimation/map data creation unit 62 to the driving control unit 63. In addition, the map data 73 is stored in the storage 41.

The driving of the driving actuator 51 is controlled under the control of the driving control unit 63 such that the self-position PS is the target position TP. The driving control is executed in this way. The driving control is continued until the self-position PS of the radiation generation apparatus 11 is the target position TP (NO in step ST130).

In a case where the self-position PS of the radiation generation apparatus 11 is the target position TP (YES in step ST130), as shown in FIG. 9, the driving control is transitioned to the alignment control (step ST140). In this case, the radiation source 13 is set to the imaging preparation position shown in FIG. 1 or FIG. 2.

As shown in FIG. 10, the alignment control image 85 captured by the camera 32 is input to the cassette contour extraction unit 80. In the cassette contour extraction unit 80, the contour OLC of the electronic cassette 12 is extracted from the alignment control image 85 by using the cassette contour extraction model 86. The cassette contour extraction result 87 is output from the cassette contour extraction unit 80 to the alignment control unit 82.

The radiation source position/posture derivation unit 81 derives the position and the posture of the radiation source 13 based on the measurement value of the radiation source position detection sensor 53. The derivation result 88 of the position and the posture of the radiation source 13 is output from the radiation source position/posture derivation unit 81 to the alignment control unit 82.

The driving of the alignment actuator 52 is controlled under the control of the alignment control unit 82 such that the radiation source 13 and the electronic cassette 12 face each other. The alignment control is executed in this way. The alignment control is continued until the radiation source 13 and the electronic cassette 12 face each other (NO in step ST150).

In a case where the radiation source 13 and the electronic cassette 12 face each other (YES in step ST150), the irradiation switch can be operated. The operator OP operates the irradiation switch to issue an instruction to start irradiation. As a result, the radiation R is emitted from the radiation source 13, and the radiographic image 18 is output by the electronic cassette 12 (step ST160).

As described above, the radiation generation apparatus 11 includes the body part 14 including the radiation source 13 that emits the radiation R toward the patient P, and the body part 14 can autonomously drive by the carriage unit 16 having the wheels 15. The driving control unit 63 executes the driving control of the carriage unit 16 to the target position TP based on the surrounding environment and the self-position PS recognized by the driving control image 70. The alignment control unit 82 executes the alignment control of the radiation source 13 and the electronic cassette 12 based on the electronic cassette 12 recognized by the alignment control image 85. That is, the driving control is executed based on the surrounding environment and the self-position PS recognized by the driving control image 70, but the alignment control is executed based on the electronic cassette 12 recognized by the alignment control image 85. Furthermore, in the driving control and the alignment control, the target as the control indicator is switched from the relatively macroscopic surrounding environment to the relatively microscopic electronic cassette 12. Therefore, it is possible to accurately perform not only the driving control to the target position TP but also the alignment control between the radiation source 13 and the electronic cassette 12.

The radiation source 13 and the electronic cassette 12 are disposed at a distance corresponding to the SID. Therefore, in a case where the posture of the radiation source 13 and the electronic cassette 12 deviates by, for example, 1°, the shift between the irradiation center RC of the radiation R and the center CC of the detection surface 17 of the electronic cassette 12 is an amount that cannot be ignored. Therefore, an effect that the alignment control between the radiation source 13 and the electronic cassette 12 can be accurately performed is extremely useful.

As shown in FIG. 9, the processor 40 transitions from the driving control to the alignment control in a case where the self-position PS is the target position TP. Therefore, it is possible to transition from the driving control to the alignment control at an appropriate timing. The alignment control can be executed without any problem.

As shown in FIGS. 7 and 8, in the driving control, the map data 73 of the surrounding environment is created while estimating the self-position PS from the driving control image 70 captured by the camera 33. Therefore, the self-position PS can be estimated with high accuracy. The autonomous driving can be performed even in an unknown environment. In addition, the target position TP can be reached on an appropriate driving path such as a shortest path and a path avoiding an obstacle.

As shown in FIG. 10, in the alignment control, the contour OLC of the electronic cassette 12 is extracted from the alignment control image 85 captured by the camera 32. Therefore, it is possible to contribute to more accurate alignment between the radiation source 13 and the electronic cassette 12.

As shown in FIGS. 10 and 11, in the alignment control, the cassette contour extraction model 86 trained by the alignment-control training image 85L including the electronic cassette 12 is used. Therefore, the contour OLC of the electronic cassette 12 can be easily extracted from the alignment control image 85.

The body part 14 includes the arm 27 that holds the radiation source 13 and can change the position and the posture of the radiation source 13. As shown in FIGS. 1 to 3, the radiation source 13 is movable between the accommodation position and the imaging preparation position by the arm 27. As shown in FIG. 9, the processor 40 sets the radiation source 13 to the accommodation position while executing the driving control, and sets the radiation source 13 to the imaging preparation position from the accommodation position in a case of transitioning from the driving control to the alignment control. Therefore, the radiation source 13 does not interfere with the autonomous driving. In addition, the alignment control can be executed without any problem.

### Second Embodiment

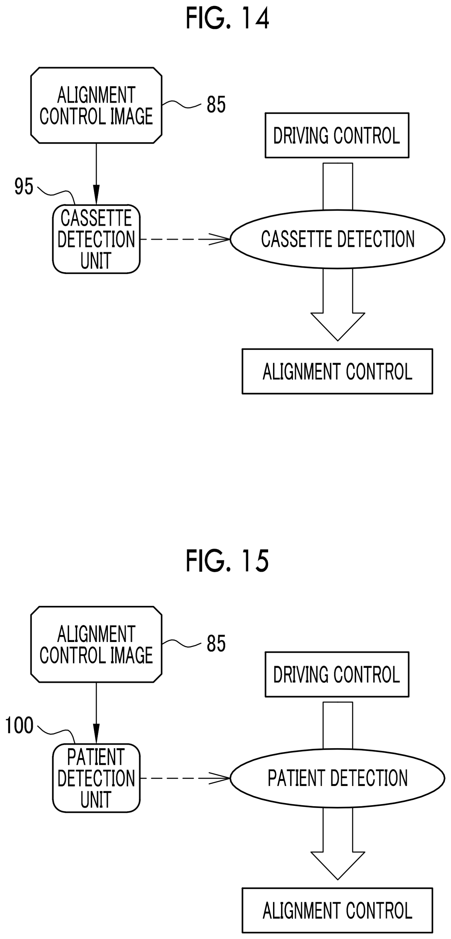

In the first embodiment, the driving control is transitioned to the alignment control in a case where the self-position PS is the target position TP, but the present disclosure is not limited to this. As an example, the second embodiment may be as shown in FIGS. 14 and 15. It should be noted that, in the present embodiment, the radiation source 13 is set to the imaging preparation position from the accommodation position in a case where the driving control is started and the standby position HP is left.

As shown in FIG. 14, the processor 40 of the present embodiment functions as a cassette detection unit 95 in addition to each processing unit of the first embodiment. The alignment control image 85 is input to the cassette detection unit 95. The cassette detection unit 95 detects whether or not the electronic cassette 12 is shown in the alignment control image 85 by using an image recognition technology.

In a case where it is detected by the cassette detection unit 95 that the electronic cassette 12 is shown in the alignment control image 85, the processor 40 of the present embodiment transitions from the driving control to the alignment control. In a case where it is detected that the electronic cassette 12 is shown in the alignment control image 85, it is an example of a "case where a set condition is satisfied" according to the disclosed technology. It should be noted that the cassette detection unit 95 may detect whether or not the electronic cassette 12 is shown in the alignment control image 85 by using the trained model such as the cassette contour extraction model 86 of the first embodiment.

Alternatively, as shown in FIG. 15, the processor 40 of the present embodiment functions as a patient detection unit 100 in addition to each processing unit of the first embodiment. The alignment control image 85 is input to the patient detection unit 100. The patient detection unit 100 detects whether or not the patient P is shown in the alignment control image 85 by using an image recognition technology. In a case where it is detected by the patient detection unit 100 that the patient P is shown in the alignment control image 85, the processor 40 of the present embodiment transitions from the driving control to the alignment control. In a case where it is detected that the patient P is shown in the alignment control image 85, it is an example of a "case where a set condition is satisfied" according to the disclosed technology. In a case where it is detected that the electronic cassette 12 or the patient P is shown in the alignment control image 85 in this way, the driving control can be transitioned to the alignment control at an appropriate timing, and the alignment control can be executed without any problem.

### Third Embodiment

In the first embodiment, the contour OLC of the electronic cassette 12 is extracted from the alignment control image 85, but the present disclosure is not limited to this. As an example, the third embodiment may be as shown in FIG. 16.

In FIG. 16, the processor 40 of the present embodiment functions as a joint point extraction unit 105 instead of the cassette contour extraction unit 80 of the first embodiment. The alignment control image 85 is sequentially input to the joint point extraction unit 105. The joint point extraction unit 105 extracts the joint point J of the patient P from the alignment control image 85 by using a joint point extraction model 106. FIG. 16 shows a case where both shoulder joints, both elbow joints, and both hip joints are extracted as the joint points J. According to a line connecting each joint point J, the center IC of the imaging part of the patient P and the posture of the patient P with respect to the radiation source 13 are known. The joint point extraction model 106 is a trained model configured by, for example, a convolutional neural network and the like, similarly to the cassette contour extraction model 86. The joint point extraction unit 105 outputs a joint point extraction result 107 to the alignment control unit 82.

In a case where the posture of the radiation source 13 is inclined with respect to the patient P, the alignment control unit 82 controls the driving of the alignment actuator 52 to rotate the radiation source 13 to eliminate the inclination. In addition, in a case where the irradiation center RC of the radiation R and the center IC of the imaging part of the patient P are shifted, the alignment control unit 82 controls the driving of the alignment actuator 52 to move the radiation source 13 to eliminate the shift. By extracting the joint point J of the patient P from the alignment control image 85 in this way, it is possible to contribute to more accurate alignment between the radiation source 13 and the electronic cassette 12.

### Fourth Embodiment

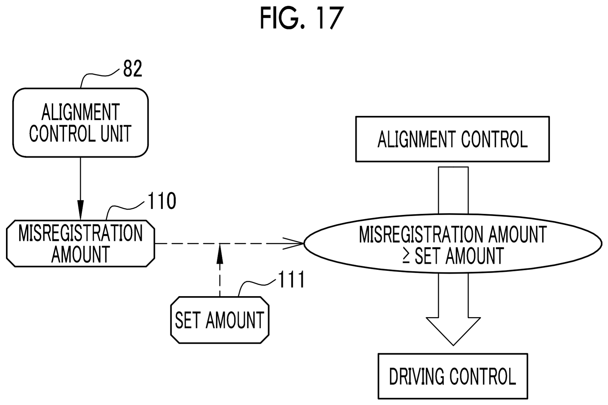

As shown in FIG. 17 as an example, in the present embodiment, the alignment control unit 82 obtains a misregistration amount 110 between the irradiation center RC of the radiation R and the center CC of the detection surface 17 of the electronic cassette 12 or between the irradiation center RC of the radiation R and the center IC of the imaging part of the patient P. The misregistration amount 110 is compared with a set amount 111. The set amount 111 is an amount of misregistration that takes, for example, 3 seconds or more to be eliminated in the alignment control. In a case where the movement amount of the radiation source 13 in the alignment control is, for example, 100 mm/sec, the set amount 111 is 100 × 3 = 300 mm.

In a case where the misregistration amount 110 is equal to or larger than the set amount 111, the processor 40 transitions from the alignment control to the driving control. That is, in a case where the misregistration amount 110 is equal to or larger than the set amount 111, the alignment by fine adjustment (alignment control) is abandoned, and the alignment by coarse adjustment (driving control) is returned to. In a case where the misregistration amount 110 is equal to or larger than the set amount 111, it is an example of a "case where a set condition is satisfied" according to the disclosed technology. In a case where the misregistration amount 110 is equal to or larger than the set amount 111, it is considered that the operator OP collides with the radiation generation apparatus 11 or the like. In this way, as a result, the alignment between the radiation source 13 and the electronic cassette 12 can be completed earlier than in a case where the alignment control is still executed even in a case where the misregistration amount 110 is equal to or larger than the set amount 111.

### Fifth Embodiment

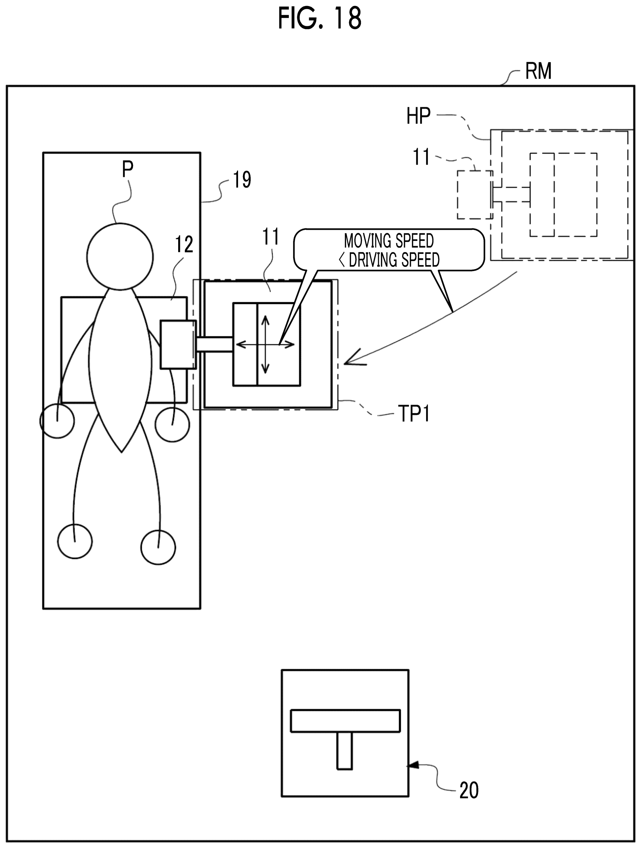

As described in the fourth embodiment, the processor 40 may move or revolve the body part 14 forward, backward, left, and right at the target position TP for fine adjustment by the carriage unit 16 while executing the alignment control. In such a case, as shown in FIG. 18 as an example, the processor 40 sets a moving speed of the body part 14 in the alignment control to be lower than a driving speed of the body part 14 in the driving control. For example, the moving speed of the body part 14 in the alignment control is set to 1/10 of the driving speed of the body part 14 in the driving control. In this way, in a case of driving a relatively long distance to the target position TP as in the driving control, it is possible to reach the target position TP without spending time. In addition, in a case of performing the alignment by fine adjustment as in the movement of the alignment control, it is possible to reduce a concern that the body part 14 does not stop at the original movement position or the body part 14 deviates from the original movement position due to inertia.

The alignment control image 85 may be displayed on the operation panel 28. In this case, the contour OLC of the electronic cassette 12, the center CC of the detection surface 17 of the electronic cassette 12, the irradiation center RC of the radiation R, and the like may be displayed in a superimposed manner on the alignment control image 85 as marks.

The carriage unit 16 may be omitted, and the wheels 15 may be directly attached to a lower portion of the body part 14.

The camera 32 may be attached to the arm 27 instead of the radiation source 13. Similarly, the camera 33 may be provided in the body part 14 instead of the carriage unit 16. A plurality of the cameras 32 and 33 may be provided.

The camera 32 and the camera 33 may be integrated into one camera. In this case, the orientation of the camera is set to an orientation in which the driving control image 70 can be captured in a case of executing the driving control, and is set to an orientation in which the alignment control image 85 can be captured in a case of executing the alignment control.

The environment information sensor is not limited to the example of the cameras 32 and 33. A light detection and ranging (LiDAR) sensor, a time-of-flight (TOF) sensor, or the like may be used. In addition, an inertial measurement unit (IMU) in which an acceleration sensor and a gyro sensor are combined, an ultrasonic sensor, a radar sensor, a magnetic sensor, or the like may be used.

The radiographic image detection device is not limited to the example of the electronic cassette 12. A computed radiography (CR) cassette may be used. In addition, the subject is not limited to the example of the patient P. The subject may be a diseased animal such as a dog or a cat.

In each of the above-described embodiments, for example, each processing of each processing unit such as the image acquisition unit 60, the feature point extraction unit 61, the self-position estimation/map data creation unit 62, the driving control unit 63, the cassette contour extraction unit 80, the radiation source position/posture derivation unit 81, the alignment control unit 82, the cassette detection unit 95, the patient detection unit 100, and the joint point extraction unit 105 is executed by any computer. In addition, any computer may execute these types of processing by a processor as hardware, a program as software, or a combination thereof. In such a case, the processor is configured to execute various types of processing in each of the above-described embodiments in cooperation with the program, and may function as each unit or each means in each of the above-described embodiments. In addition, the execution order of the processing by the processor is not limited to the above-described order and may be changed as appropriate. Any computer may be a general-purpose computer, a computer for specific use, a workstation, or another system capable of executing each processing.

The processor may be configured using one or more pieces of hardware, and the type of hardware is not limited. For example, the processor may be configured by an example CPU or micro processing unit (MPU), a programmable logic device such as a field programmable gate array (FPGA), a dedicated circuit for executing specific processing such as an application specific integrated circuit (ASIC), a graphics processing unit (GPU), a neural processing unit (NPU), or hardware. Types of hardware may be a combination of different types of hardware. In a case in which the plurality of types of hardware are configured to execute one or a plurality of types of processing of a certain processor, the plurality of types of hardware may exist in devices physically separated from each other or may exist in the same device. In any embodiment, the order of each processing via the processor is not limited to the above order and may be appropriately changed. The hardware is configured by an electric circuit (circuitry) in which circuit elements such as semiconductor elements are combined.

The program may be software such as firmware or a microcode. For example, the program may be a program module group, and each function thereof may be implemented by a processor configured to execute each function. The program may be a program code or a plurality of code segments stored in one or a plurality of non-transitory computer-readable media (for example, a storage medium or other storage). The program may be divided and stored in a plurality of non-transitory computer-readable media present in apparatuses physically separated from each other. The program code or the code segments may represent any combination of a procedure, a function, a subprogram, a routine, a subroutine, a module, a software package, a class, an instruction, a data structure, and a program statement. The program code or the code segments may be connected to other code segments or hardware circuits by transmitting and receiving information, data, an argument, a parameter, or content of a memory.

The technologies according to the following supplementary notes can be understood from the above description.

### [Supplementary Note 1]

A radiation generation apparatus, in which a body part including a radiation source that emits radiation toward a subject is autonomously drivable by a driving mechanism having wheels, the radiation generation apparatus comprising:
an environment information sensor; and
a processor,
wherein the processor is configured to execute:
   driving control of the driving mechanism to a target position based on a surrounding environment and a self-position recognized from output data of the environment information sensor; and
   alignment control of the radiation source and a radiographic image detection device based on the radiographic image detection device or the subject recognized from the output data.

### [Supplementary Note 2]

The radiation generation apparatus according to Supplementary Note 1,
wherein the processor transitions from the driving control to the alignment control or transitions from the alignment control to the driving control in a case where a set condition is satisfied.

### [Supplementary Note 3]

The radiation generation apparatus according to Supplementary Note 2,
wherein the processor transitions from the driving control to the alignment control in a case where the self-position is the target position.

### [Supplementary Note 4]

The radiation generation apparatus according to Supplementary Note 2,
wherein the processor transitions from the driving control to the alignment control in a case where the radiographic image detection device or the subject is detected from the output data.

### [Supplementary Note 5]

The radiation generation apparatus according to any one of Supplementary Notes 2 to 4,
wherein the processor transitions from the alignment control to the driving control in a case where a misregistration amount between the radiation source and the radiographic image detection device or the subject is equal to or larger than a set amount.

### [Supplementary Note 6]

The radiation generation apparatus according to any one of Supplementary Notes 1 to 5,
wherein, in the driving control, map data of the surrounding environment is created while estimating the self-position from the output data.

### [Supplementary Note 7]

The radiation generation apparatus according to any one of Supplementary Notes 1 to 6,
wherein, in the alignment control, a contour of the radiographic image detection device is extracted from the output data.

### [Supplementary Note 8]

The radiation generation apparatus according to Supplementary Note 7,
wherein, in the alignment control, a trained model trained by output data for training including the radiographic image detection device is used.

### [Supplementary Note 9]

The radiation generation apparatus according to any one of Supplementary Notes 1 to 6,
wherein, in the alignment control, a joint point of the subject is extracted from the output data.

### [Supplementary Note 10]

The radiation generation apparatus according to any one of Supplementary Notes 1 to 9,
wherein the body part includes an arm that holds the radiation source and is capable of changing a position and/or a posture of the radiation source,
the radiation source is movable between an accommodation position and an imaging preparation position by the arm, and
the processor is configured to:
   set the radiation source to the accommodation position while executing the driving control; and
   set the radiation source from the accommodation position to the imaging preparation position in a case of transitioning from the driving control to the alignment control.

### [Supplementary Note 11]

The radiation generation apparatus according to any one of Supplementary Notes 1 to 10,
wherein the alignment control includes control of moving the body part at the target position by the driving mechanism, and
a moving speed of the body part in the alignment control is lower than a driving speed of the body part in the driving control.

The technology of the present disclosure can also be combined with various embodiments and/or various modification examples described above, as appropriate. In addition, it goes without saying that the present disclosure is not limited to each of the embodiments described above, various configurations can be adopted as long as the configuration does not deviate from the gist. Furthermore, the technology of the present disclosure extends to a storage medium that non-transitorily stores the program, and a computer program product including the program, in addition to the program.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In addition, in order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". That is, "A and/or B" means that it may be only A, only B, or a combination of A and B. In addition, in the present specification, also in a case where three or more matters are expressed in association by "and/or", the same concept as "A and/or B" is applied.

All of the documents, the patent applications, and the technical standards described in the present specification are incorporated herein by reference to the same extent as in a case where each of the documents, patent applications, and technical standards is specifically and individually described by being incorporated by reference.

### Explanation of References

10: radiography system
11: radiation generation apparatus
12: electronic cassette
13: radiation source
14: body part
15: wheel
16: carriage unit
17: detection surface
18: radiographic image
19: decubitus imaging table
20: upright imaging table
21: holder
22: support column
25: front part
26: rear part
27: arm
28: operation panel
30: radiation tube
31: irradiation field limiter
32, 33: camera
40: processor
41: storage
42: operation program
43: control data
50: communication I/F
51: driving actuator
52: alignment actuator
53: radiation source position detection sensor
60: image acquisition unit
61: feature point extraction unit
62: self-position estimation/map data creation unit
63: driving control unit
70: driving control image
71: feature point extraction result
71P: feature point extraction result (past result)
72: estimation result
73: map data
73P: map data (past data)
80: cassette contour extraction unit
81: radiation source position/posture derivation unit
82: alignment control unit
85: alignment control image
85L: alignment-control training image
86: cassette contour extraction model
87: cassette contour extraction result
87CA: ground-truth data
87L: training cassette contour extraction result
88: derivation result
90: learning data
95: cassette detection unit
100: patient detection unit
105: joint point extraction unit
106: joint point extraction model
107: joint point extraction result
110: misregistration amount
111: set amount
CC: center of detection surface of electronic cassette
F: frame
FP: feature point
HP: standby position
IC: center of imaging part
J: joint point
OLC: contour of electronic cassette
OP: operator
P: patient
PS: self-position
R: radiation
RC: irradiation center of radiation
RM: imaging room
ST100, ST110, ST120, ST130, ST140, ST150, ST160: step
TP: target position
TP1, TP2: first target position, second target position

## Claims

1. A radiation generation apparatus (11), in which a body part (14) including a radiation source (13) that emits radiation toward a subject is autonomously drivable by a driving mechanism having wheels (15), the radiation generation apparatus comprising:
an environment information sensor (32, 33); and
a processor (40),
wherein the processor is configured to execute:
driving control of the driving mechanism to a target position based on a surrounding environment and a self-position recognized from output data of the environment information sensor (32, 33); and
alignment control of the radiation source and a radiographic image detection device (12) based on the radiographic image detection device or the subject recognized from the output data.

2. The radiation generation apparatus according to claim 1,
wherein the processor (40) transitions from the driving control to the alignment control or transitions from the alignment control to the driving control in a case where a set condition is satisfied.

3. The radiation generation apparatus according to claim 2,
wherein the processor (40) transitions from the driving control to the alignment control in a case where the self-position is the target position.

4. The radiation generation apparatus according to claim 2,
wherein the processor transitions (40) from the driving control to the alignment control in a case where the radiographic image detection device (12) or the subject is detected from the output data.

5. The radiation generation apparatus according to claim 2,
wherein the processor transitions from the alignment control to the driving control in a case where a misregistration amount between the radiation source (13) and the radiographic image detection device (12) or the subject is equal to or larger than a set amount.

6. The radiation generation apparatus according to claim 1,
wherein, in the driving control, map data of the surrounding environment is created while estimating the self-position from the output data.

7. The radiation generation apparatus according to claim 1,
wherein, in the alignment control, a contour of the radiographic image detection device is extracted from the output data.

8. The radiation generation apparatus according to claim 7,
wherein, in the alignment control, a trained model trained by output data for training including the radiographic image detection device (12) is used.

9. The radiation generation apparatus according to claim 1,
wherein, in the alignment control, a joint point of the subject is extracted from the output data.

10. The radiation generation apparatus according to claim 1,
wherein the body part (14) includes an arm (27) that holds the radiation source and is capable of changing a position and/or a posture of the radiation source,
the radiation source (13) is movable between an accommodation position and an imaging preparation position by the arm, and
the processor is configured to:
set the radiation source (13) to the accommodation position while executing the driving control; and
set the radiation source from the accommodation position to the imaging preparation position in a case of transitioning from the driving control to the alignment control.

11. The radiation generation apparatus according to claim 1,
wherein the alignment control includes control of moving the body part at the target position by the driving mechanism, and
a moving speed of the body part (14) in the alignment control is lower than a driving speed of the body part in the driving control.

12. An operation method of a radiation generation apparatus (11), in which a body part (14) including a radiation source (13) that emits radiation toward a subject is autonomously drivable by a driving mechanism having wheels (15), the operation method comprising:
executing driving control of the driving mechanism to a target position based on a surrounding environment and a self-position recognized from output data of an environment information sensor (32, 33); and
executing alignment control of the radiation source (13) and a radiographic image detection device (12) based on the radiographic image detection device or the subject recognized from the output data.

13. A computer-readable storage medium storing an operation program of a radiation generation apparatus, in which a body part (14) including a radiation source (13) that emits radiation toward a subject is autonomously drivable by a driving mechanism having wheels (15), the operation program causing a computer to execute a process comprising:
executing driving control of the driving mechanism to a target position based on a surrounding environment and a self-position recognized from output data of an environment information sensor (32, 33); and
executing alignment control of the radiation source (13) and a radiographic image detection device (12) based on the radiographic image detection device or the subject recognized from the output data.
